(19) Europäisches Patentamt
European Patent Office
Office européen des brevets

(11) Publication number : **0 633 031 A1**

(12) **EUROPEAN PATENT APPLICATION**

(21) Application number : **94304571.6**

(51) Int. Cl.[6] : **A61L 27/00**

(22) Date of filing : **23.06.94**

(30) Priority : **24.06.93 US 82219**

(43) Date of publication of application : **11.01.95 Bulletin 95/02**

(84) Designated Contracting States : **CH DE DK FR GB IT LI NL SE**

(71) Applicant : **SIEMENS AKTIENGESELLSCHAFT**
**St Martin Strasse 76**
**D-81541 München (DE)**

(72) Inventor : **Vachon, David**
**16107 Harvest Street**
**Granada Hills, CA 91344 (US)**

(74) Representative : **Rees, David Christopher et al**
**Kilburn & Strode**
**30 John Street**
**London WC1N 2DD (GB)**

(54) **Compound and method of applying anti-fouling coatings on medical devices.**

(57) A composition which inhibits the *in vivo* formation of scar tissue at a surface of a device coated with the composition when the device is inserted at an *in vivo* site. The composition promotes growth of viable tissue at the site of insertion of a device and comprises a biocompatible polymer and an extracellular matrix molecule or fragment thereof.

EP 0 633 031 A1

The present invention relates to compositions and their use for modifying the biocompatibility of the surfaces of medical devices to prevent fouling of these devices.

The development of implantable medical devices, such as artificial blood vessels, catheters, pacemakers, prosthetic implants, and drug delivery systems for the controlled release of therapeutics, represent a small fraction of material-dependent devices in a rapidly developing area. However, the long-term use of these implantable devices can be impaired by thrombosis, infection, as well as chronic inflammation, generally considered as fouling of these devices. The fouling can result in a decrease in device performance and longevity.

Proteins deposited from the blood can initiate deposition of the cellular components within the blood onto the surface of the medical devices. This deposition constricts and hinders the use of devices such as artificial blood vessels. Inflammation, a natural response to an implant, can also initiate reactions that impede the function of devices such as ophthalmic lenses and pacemakers leads. Consequently, it is highly desirable to prevent such fouling of biomaterial surfaces.

Fouling of a medical device can be prevented by modifying the surface of the device to inhibit fouling. Many different materials can be used to limit and inhibit fouling. One such material is polyethylene oxide (PEO). However, there are several problems associated with PEO coatings.

A major problem with PEO is its high solubility in water. This necessitates the use of complex and expensive methods allowing the utilization of PEO as an anti-fouling coating material. These complex techniques include such methods as: covalent grafting; plasma polymerization; preparation of polymers containing pendant groups; preparation of block co-polymers; and the absorption of surfactants into a polymeric surface. The complexity of these techniques raises the level of difficulty in controlling coating uniformity, thickness and consistency.

Furthermore, many of these reactions use catalysts and/or reaction initiators that result in undesirable reaction byproducts which have to be subsequently removed by separation techniques. For example, U.S. Patent No. 4,177,056 describes the use of water insoluble PEO's terminated with bismethacrylates. These require a free radical initiator for polymerization, resulting in undesirable byproduct contaminants within the coating.

Thus, many current techniques of applying surface modifiers are disadvantageous because of their complexity, cost, lack of consistency, uniformity and reduced effectiveness in protein repulsion.

Accordingly, there is a need for non-toxic compositions and methods of applying these compositions to medical devices to render surfaces of the medical devices biocompatible, where the compositions and methods are simple and provide consistent and uniform thicknesses.

According to the present invention, there is provided a composition for coating a surface of an implantable device, wherein the composition inhibits formation of scar tissue at an *in vivo* site when the device is inserted at the site, characterised in that the composition comprises a biocompatible polymer and an extracellular matrix molecular or fragment thereof.

The invention also extends to the use of such a composition in the inhibition of scar tissue formation at an *in vivo* site of insertion of a device, in which a surface of the device is coated with the composition.

The present invention provides compositions of matter and methods for their application that satisfy these needs. Coatings prepared in accordance with the present invention have high anti-fouling characteristics and have excellent tenacity and <u>lubricity</u>. Furthermore, the method of affixing the compositions onto the surfaces of medical devices is simple, results in uniform and consistent coatings, and does not use added catalysts or reaction initiators.

A method suitable for preparing these coatings comprises the following steps: 1) coating at least a portion of a medical device with an aqueous solution of a photochemically reactive or thermochemically reactive polymer comprising at least one linear poly(alkylene oxide) segment having at least two repeating alkylene oxide groups, substantially all of the polymer having at least two terminal photochemically reactive or thermochemically reactive groups, the molecular weight of the polymer being at least 10,000 and sufficiently low that the polymer is soluble in water; and 2) curing the coating by exposing the coating to ultraviolet light in case of a photochemically reactive polymer or by heating the polymer in the case of a thermochemically reactive polymer.

By the term "poly(alkylene) oxide segment" it is meant a segment or unit having the structure

$$-\left[ R - O \right]_n-$$

where $R_1$ is an alkyl moiety containing at least 1 carbon atom and n is at least 2; typically n is at least 16.

The reactive polymer used for these coatings typically is derived from an aliphatic poly(ether glycol), referred to herein as a poly(alkylene oxide), such as poly(ethylene oxide) or poly(ethylene glycol). It must be understood that poly(alkylene oxides) and poly(alkylene glycols) are both hydroxy terminated aliphatic polyethers,

the only difference being in molecular weight. Polymers with molecular weights of less than 10,000 are referred to herein as poly(alkylene glycols), and those with molecular weights of 10,000 and above are referred to as poly(alkylene oxides).

The formula of the reactive polymer depends on the structure of the original polymer and the type of photochemically reactive group that is used. When a hydroxy terminated alkylene oxide polymer is used, the reactive polymer has the formula:

$$(1) \qquad P_1-O-\left[R_1-O\right]_n-R_1-O-P_1$$

When an amine terminated poly(alkylene oxide) is used, the reactive polymer has the formula:

$$(2) \qquad P_1-\overset{R4}{\overset{|}{N}}-R_1-O-\left[R_1-O\right]_n-\overset{R4}{\overset{|}{N}}-P_1$$

When penta-erythritol is used as building block, the reactive polymer can have one of the formulas:

$$(3) \qquad C-\left[-CH_2-O-\left(-R_1-O-\right)_n-R_1-O-P_1\right]_4$$

$$(4) \qquad C-\left[-CH_2-\underset{R_4}{\underset{|}{N}}-R_1-\left(-O-R_1-\right)_n-O-R_1-\underset{R_4}{\underset{|}{N}}-P_1\right]_4$$

When a diethanol amine or triethanol amine is used as a building block, the reactive polymer has the formula:

$$(5) \qquad R_5-N-\left[-R_6-O-\left(-R_1-O-\right)_n-R_1-O-P_1\right]_x$$

In formulas 1-5:

$R_1$ is an alkyl group containing at least one carbon atom and typically 2 to 10 carbon atoms;

n is at least 1 and sufficiently large that the average molecular weight of the polymer is at least 10,000, and preferably about 20,000;

$R_4$ is selected from the group consisting of hydrogen, hydrocarbon groups containing up to about 10 carbon atoms, and heteroatom-containing hydrocarbon groups containing up to about 10 carbon atoms;

$R_6$ is an alkyl group containing up to 10 carbon atoms, and typically 2 carbon atoms;

where x is 2 or 3, and for x of 2, $R_5$ is selected from the group consisting of hydrogen, hydrocarbon groups containing up to about 10 carbon atoms, and heteroatom-containing hydrocarbon groups containing up to about 10 carbon atoms, and for x of 3, $R_5$ is excluded;

$P_1$ is a photochemically reactive or thermochemically reactive moiety capable of undergoing cross-linking on photoactivation or heating respectively, to form a hydrophilic, lubricous and tenacious coating which resists fouling.

Each $P_1$ can be the same or different, but for simplicity, the $P_1$'s are the same. Preferably $P_1$ is photochemically reactive.

The term "heteroatom-containing hydrocarbon group" refers to a hydrocarbon group that also contains a

heteroatom such as oxygen, nitrogen, or sulfur.

Preferably $R_4$ is hydrogen or a lower alkyl group having from 1 to about 6 carbon atoms, and most preferably is hydrogen or methyl.

The base polymer can also have different alkyl groups in blocks within its structure, such that the photochemically reactive polymer, has one of the formulas:

(6) $$P_1 - O - R_1 \left[ O - R_1 \right]_m O \left[ R_2 - O \right]_n P_1$$

(7) $$P_1 - \underset{}{\overset{R_4}{\overset{|}{N}}} - R_1 \left[ O - R_1 \right]_m O \left[ R_2 - O \right]_n R_2 - \overset{R_4}{\overset{|}{N}} - P_1$$

(8) $$C-\left[-CH_2-O-\left(-R_1-O-\right)_m-R_1-O-\left(-R_2-O-\right)_n-R_2-O-P_1\right]_4$$

(9) $$C-\left[-CH_2-\underset{R_4}{\underset{|}{N}}-R_1-\left(-O-R_1-\right)_m-O-\left(-R_2-O-\right)_n-R_2-\underset{R_4}{\underset{|}{N}}-P_1\right]_4$$

(10) $$R_5-N-\left[-R_6-O-\left(-R_1-O-\right)_m-R_1-O-\left(-R_2-O-\right)_n-R_2-O-P_1\right]_x$$

In formulas 6-10:

$R_1$ and $R_2$ are alkyl groups containing at least one carbon atom, and typically 2 to 10 carbon atoms, $R_1$ is different from $R_2$;

$P_1$, $R_4$, $R_5$, $R_6$ and x are as defined above; and

m and n are at least 1, and m and n are sufficiently large that the average molecular weight of the polymer is at least 10,000.

The photochemically reactive group typically can be a substituted benzophenone or an acidonitrophenyl group. These respectively have the structures:

(11)

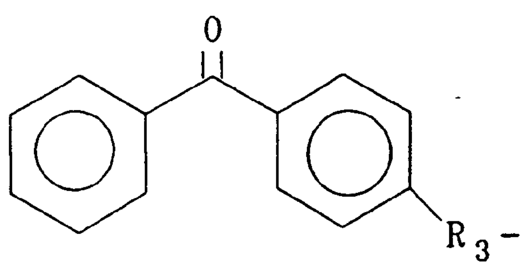

where $R_3$ is an alkyl or heteroatom-containing alkyl group having from 1 to about 6 carbon atoms, as well as

acyl groups, or alkyl or heteroatom-containing alkyl groups with acyl termini.

(12) 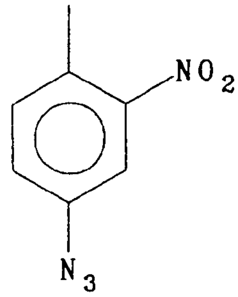

For the preparation of biodegradable coatings, it is desirable to include in the polymer backbone a moiety that renders the coating biodegradable. A polymer containing such a moiety can have one of the following formulas:

(13) $$P_1 - Y - O - [R_1 - O]_n - R_1 - O - Y - P_1$$

(14) 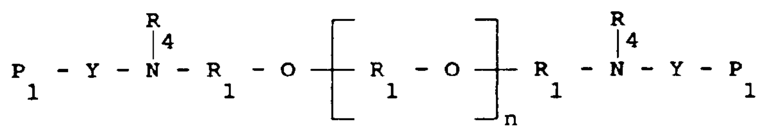

(15) $$C[ CH_2\text{-}O\text{-}( \text{-}R_1\text{-}O\text{-})_n\text{-}R_1\text{-}O\text{-}Y\text{-}P_1]_4$$

(16) 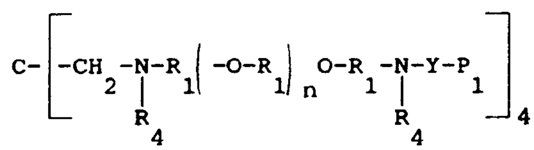

(17) $$R_5\text{-}N[R_6\text{-}O\text{-}( \text{-}R_1\text{-}O\text{-})_n\text{-}R_1\text{-}O\text{-}Y\text{-}P_1]_x$$

(18) $$P_1 - Y - O - [R_1 - O]_m - R_1 - O - [R_2 - O]_n - R_2 - O - Y - P_1$$

(19) 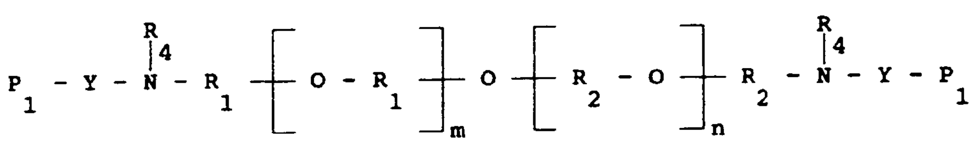

(20) $$C\text{-}\left[\text{-}CH_2\text{-}O\text{-}\left(\text{-}R_1\text{-}O\text{-}\right)_m\text{-}R_1\text{-}O\text{-}\left(\text{-}R_2\text{-}O\text{-}\right)_n\text{-}R_2\text{-}O\text{-}Y\text{-}P_1\right]_4$$

(21) $$C-\left[-CH_2\underset{\displaystyle R_4}{N}-R_1-\left(-O-R_1-\right)_m-O-\left(-R_2O\right)_n-R_2-\underset{\displaystyle R_4}{N}-Y-P_1\right]_4$$

(22) $$R_5-N-\left[-R_6O-\left(-R_1-O-\right)_m-R_1-O-\left(-R_2-O-\right)_n-R_2O-Y-P_1\right]_x$$

In formulas 13-22, each Y is independently selected from the group consisting of amino acids and peptides, and $P_1$, $R_1$, $R_2$, $R_4$, $R_5$, $R_5$ and x are as defined above; and m and n are at least 1; and m and n are sufficiently large that the molecular weight of the polymer is at least 10,000.

Amine terminated versions of Formulas 5, 10 and 22 can also be used, i.e., Formulas 5, 10, and 22 can be modified by replacing the terminal oxygens proximate to $P_1$ with N-$R_4$.

These and other features of the present invention will become better understood from the following description and appended claims.

The present invention is directed to compositions suitable for forming anti-fouling coatings on medical devices, and the method of their use. These compositions can be synthesized from a mixture of:

(a) a poly(alkylene oxide) based polymer, such as polyethylene oxide, terminated with hydroxy or amine groups, and having a molecular weight of at least 10,000;

(b) a precursor of a photochemically reactive or thermochemically reactive moiety such as a halogenated methylbenzophenone or a halogenated acidonitrophenyl;

(c) a strong base such as sodium hydride; and

(d) a non-reactive organic solvent such as tetrahydrofuran or toluene.

The term "non-reactive" indicates that the solvent will not deleteriously react with any of the components of the polymerization reaction.

The poly(alkylene oxide) is modified by alkylation or acylation with a reactive compound to yield a difunctional poly(alkylene oxide). This modified polymer is then applied on a medical device. When the coating is exposed to ultraviolet light or heat, the modified polymer polymerizes and crosslinks to form an anti-fouling coating.

## 1. Poly(alkylene oxide) Based Polymer

Poly(alkylene oxide) is a generic name given to a class of materials which contain an alkylene oxide repeat unit. The repeating unit can be methylene, ethylene, propylene, butylene, and any other hydrocarbon containing at least one carbon atom, and typically can contain up to about 10 carbon atoms.

For example, the poly(alkylene oxide) based compound can be a polyethylene oxide, having an ethylene oxide repeat unit, with the ends terminated by hydroxy groups. An exemplary compound has the structure:

(23) $$H-O-\left[CH_2-CH_2-O\right]_n-CH_2-CH_2-OH$$

The poly(alkylene oxide) can also be terminated at both ends with amine ($NH_2$) moiety instead of a hydroxy moiety. An exemplary compound has the structure:

(24) $$H_2N-\left[\overset{\displaystyle H}{\underset{\displaystyle H}{C}}-\overset{\displaystyle H}{\underset{\displaystyle H}{C}}-O\right]_n-CH_2-CH_2-NH_2$$

Penta-erythritol or its derivatives can be used as a building block to produce a modified polymer. An exemplary compound based on penta-erythritol has the formula:

(25) $$C-\left[CH_2O-\left(CH_2-CH_2-O\right)_n-P_1\right]_4$$

Secondary and tertiary amines can be used as a building block to produce poly(alkylene oxide) based polymers. Exemplary of such compound is:

(26) 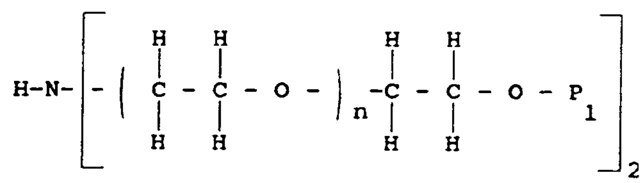

In Formulas 23-26, n is sufficiently large that the average molecular weight of the polymer is at least 10,000.

Preferably the poly(alkylene oxide) based polymer has an average molecular weight of less than 100,000, a preferred molecular weight being approximately 20,000.

The poly(alkylene oxide) based polymer can contain more than one type of alkyl group, such as the class of materials known as POLOXAMERS™, having the formula:

(27) $$HO-\left[R_1-O\right]_m-R_1-O-\left[R_2-O\right]_n-R_2-O-H$$

These poly(alkylene oxide) based polymers containing two or more blocks of alkylene oxide groups can be used to produce the modified polymers of Formulas 6-10 and 18-22.

Amine terminated poly(alkylene oxides) containing more than one type of alkyl group also can be used, represented by the formula:

(28) 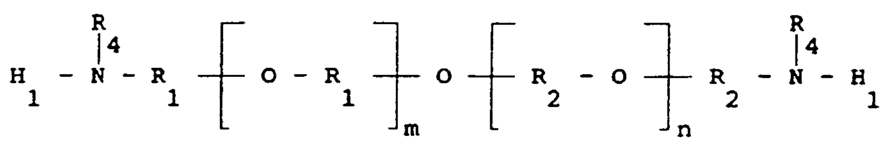

In Formulas 27-28, $R_1$ and $R_2$ are alkyl moieties, containing at least one carbon atom, $R_1$ is different from $R_2$, n is at least 1 and m is at least 1, $R_4$ is as defined above, and n and m are sufficiently large that the average molecular weight of the polymer is at least 10,000. For example, the structure of the polymer depicted in Formula 27 can correspond to a poly(oxyethylene-oxypropylene), where the ethylene to propylene ratio is from about 1:1 to about 3:1.

An example of amine terminated poly(alkylene oxides) suitable for preparing the polymers of Formulas 7, 24 and 28, are marketed under the tradename "JEFFAMINE" by Texaco Chemical Co., Houston, Texas.

2. The Reactive Compound $P_1$

The reactive group is typified by thermochemical groups and photochemical groups, as described and exemplified in U.S. Patent No. 3,959,078 (Guire), the teachings of which are incorporated herein by reference.

The photochemically reactive groups (the covalent bonding of which are activated by actinic radiation) can be aryl, alkyl and acyl azides, oxazidines, isocyanates (nitrene generators), alkyl and ketodiazo derivatives and diazirines (carbene generators), aromatic ketones (triplet oxygen generators), aromatic diazonium derivatives and numerous classes of carbonium ion and radical generators. Reference is made to Frederick J. Darfler and Andrew M. Tometsko, Chapter 2 of Chemistry and Biochemistry of Amino Acids, Peptides and Proteins (Boris Weinstein, ed) Vol. 5, Marcel Dekker, Inc. New York, 1978, for further description of photochemically reactive groups. Azidonitrophenyls, fluoroazido nitrobenzenes, and aromatic ketones form a preferred group due to their stability to chemical reaction conditions in the dark and their susceptibility to activation by light of wave lengths harmless to most biomaterials, to form short-lived reactive intermediates capable of forming covalent bonds in useful yield with most sites on the biomaterial.

Nitrophenylazide derivatives appropriate for use as photochemically reactive groups for the most part can be derived from fluoro-2-nitro-4-azidobenzene, and include 4-azido-2-nitrophenyl(ANP)-4-aminobutyryl, ANP-6-aminocaproyl, ANP-11-aminoundecanoyl, ANP-glycyl, ANP-aminopropyl, ANP-mercaptoethylamino, ANP-diaminohexyl, ANP-diaminopropyl, and ANP-polyethylene glycol. (ANP-6-aminocaproyl, ANP-Il-aminoundecanoyl, and ANP-polyethylene glycol are preferred.) Aromatic ketones preferred for use as photochemically reactive groups include benzylbenzoyl and nitrobenzylbenzoyl.

Thermochemical reactive groups (that are activated by heat energy) appropriate for use, are typified by and include diene-dienophile combinations, carbonyl groups, hydroxyl groups, amino groups (1°, 2°), epoxides, thiols, isocyanates, halides, imidates, isothiocyonates, acetylenes, maleimides, diazo compounds, and salts of sulfonyl hydrazones.

The reactive compound can be photochemically reactive, or thermochemically reactive, with photochemically reactive preferred. The photochemically reactive group allows the modified polymer to crosslink upon exposure to ultraviolet radiation.

A preferred precursor for the photochemically reactive compound is halogenated methylbenzophenone having the formula:

(29)

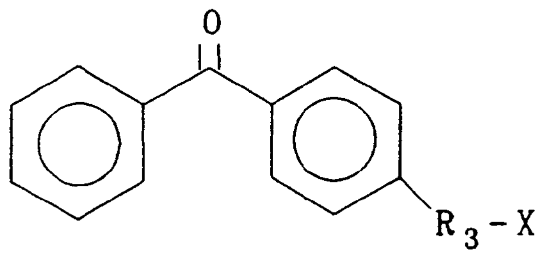

where $R_3$ is an alkyl group or alkyl group having from 1 to about 6 carbon atoms, and X is a halogen moiety.

Halogenated acidonitrophenyl can also be used, having the structure:

(30)

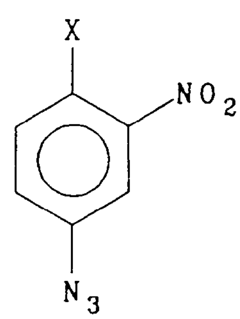

where X is a halogen moiety.

The photochemically reactive groups are incorporated into the polymer by alkylation or acylation to yield a multifunctional poly(alkylene oxide), which upon exposure to ultraviolet light undergoes cross-linking to form a tenacious, lubricous, hydrophilic, anti-fouling material.

During the alkylation process, the halogen moiety is displaced from the halogenated compounds of Formulas 15 and 16, so that the polymer contains the reactive groups, $P_1$, indicated by Formulas 5 and 6.

3. Strong Base

The reactivity of the poly(alkylene oxide) is modified by the addition of a strong base to generate a more nucleophilic species. Examples of these bases include sodium hydride, sodium hydroxide, potassium hydride, and potassium hydroxide.

4. Organic Solvent

A number of organic solvents, inert to reactants, can be used as a medium for the reaction. Solvents such as tetrahydrofuran, toluene, glyme and diglyme can be used.

5. Surfaces for Application

The solid surface that is rendered biocompatible in accordance with the invention desirably is of a synthetic or natural material that is insoluble in physiological fluids. The surface can be one or more surfaces of devices intended to function in contact with tissue and/or fluids of living organisms. The solid surface of the device can be any suitable metal such as polished titanium or stainless steel; a polymer such as polyurethane, silicone elastomers, polyethylene, polytetrafluoroethylene, poly (p-phenyleneterephthalamide), polyvinyl chloride, polypropylene, polyesters, polyacrylates (including polymethacrylates); minerals or ceramics such as hydroxyapatite; human tissue such as bone; skin and teeth; organic materials such as wood, cellulose and compressed carbon; and other natural and synthetic materials such as glass, rubber, wood and the like. Examples of devices which can be provided with biocompatible surfaces in accordance with this invention include vascular graft tubing, dialysis tubing or membrane, blood oxygenator tubing or membrane, ultrafiltration membrane, intra aortic balloons, blood bags, catheters, suture materials, soft or hard tissue prostheses, synthetic prostheses, artificial organs, and lenses for the eye such as contact and intraocular lenses.

The solid surface is desirably thermochemically unreactive. "Thermochemically unreactive" means that the surface is free of any surface treatment designed to increase the ability of the surface to thermochemically react. Examples of thermochemically unreactive surfaces include polytetrafluroethylene, polyethylene, polypropylene, polyvinyl chloride, polyvinylpyrrolidone, silicone elastomers, stainless steel and titanium.

6. Biodegradable Coatings

For the preparation of biodegradable coatings, it is desirable to incorporate in the polymer backbone, a moiety that allows hydrolysis of the coating. Suitable ingredients include amino acids such as alanine, valine, leucine, proline, methionine, aspartic acid, threonine, serine, glutamic acid, glycine, cysteine, phenylalanine, lysine, histidine, argine and aminobutyric acid. Peptide sequences such as arginine-glycine-aspartic acid can also be used.

The biodegradable coating can be used to allow the slow release of a contained drug or other therapeutic agent. Exemplary of such agents are an antimicrobial agent such as penicillin; an anti-thrombogenic agent such as heparin; an anti-inflammatory agent such as dexamethasone sodium phosphate; and a variety of enzymes.

Formulas 7, 8, 9 and 10 show polymers suitable for forming biodegradable coatings.

7. Structures of Modified Polymers

When a hydroxy-terminated poly(alkylene oxide) is substituted with a photochemically or thermochemically reactive moiety, the modified polymer can have the structure shown by Formulas 1 and 6 above. An exemplary modified polymer has the structure:

(31)

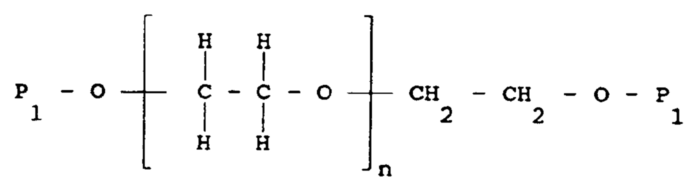

When an amine-terminated poly(alkylene oxide) is reacted with a photochemically or thermochemically reactive compound, the modified polymer has the structure of Formulas 2 and 7 above. An exemplary polymer has the structure:

(32) 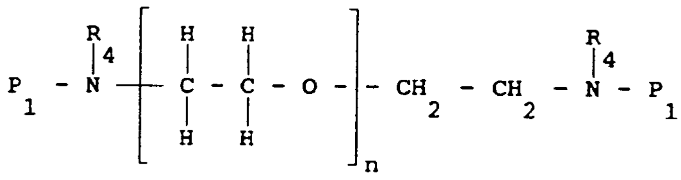

When pentaerythritol or its derivatives are used as building blocks, the modified polymer can be the structure shown by Formula 3 and 4. An exemplary pentaerythritol based polymer has the structure:

(33) $$C - \left[ CH_2O - \left( -CH_2CH_2O- \right)_n -CH_2-CH_2-O-P_1 \right]_4$$

When dialkyl amino or trialkyl amino compounds, or their derivatives are used as building blocks, poly(alkylene oxides) with multipendant or star configurations can be prepared, as shown by Formula 5.

In these formulas, n is sufficiently high that the average molecular weight of the polymer is at least 10,000, and $P_1$ is a photochemically or thermochemically reactive moiety, and $R_4$ is as defined above.

8. Preparation of Modified Polymers

The following procedure can be used to prepare modified polymers or precursors from a mixture of reactants.

A poly(ethylene oxide) polymer or other poly(alkylene oxide) based polymer is dissolved in toluene or other appropriate solvent and any water present is azeotropically removed. After water removal, dry tetrahydrofuran is added and the mixture is heated to 50 to 60°C in a dry inert atmosphere of nitrogen or argon for a sufficient time, generally about 1 hour, while stirring, to dissolve the polymer.

A strong base, preferably NaH, is then added to the polymer solution in a stoichiometric amount (two moles of base per one mole of polymer). Approximately 30 minutes later, after the dissolution of the base, a reactive group, such as photochemically reactive 4-bromomethylbenzophenone (BBE), in an amount of two moles per one mole of polymer, is added and stirred overnight.

The reaction is sampled at various intervals and the reaction sample is quenched with the acetic acid and the product equivalent weight determined by ultraviolet spectroscopy. The equivalent weight of the modified poly(alkylene oxide) samples can be calculated from corresponding ultraviolet absorbance using the expression:

$$A = cb$$

where A is the molar absorptivity, c is the concentration, and b is the path length. After quenching the reaction, the solvents are evaporated.

The residue from the reaction is then dissolved in toluene, centrifuged and filtered. The clear solvent solution is then evaporated to remove approximately 70% by volume of the toluene and the solution is then added to cold (0°C) stirred $Et_2O$ to effect precipitation. The product is then collected by filtration.

The reaction that occurs proceeds as follows:

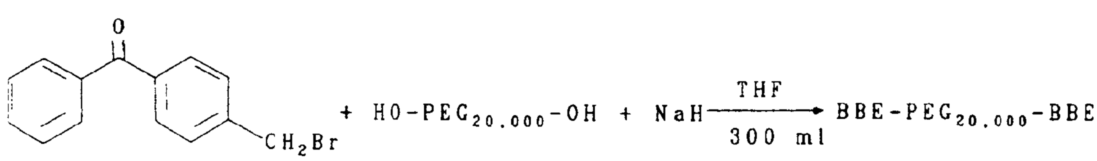

| | | | |
|---|---|---|---|
| 275 g/mole | 20,000 g/mole | 24 g/mole | 20,388 g/mole |
| 5.78 g | 140 g | 0.504 g (.63g of 80%) | 42.7 g (theory) |
| 21.0 mMole (theory) | 7.0 mMole | 21.0 mMole | 7.0 mMole |

where PEG is polyethylene glycol.

9. Applications of the Invention

Because the coating is applied as an aqueous solution followed by a thermal or photo-initiated curing or cross-linking step, the coating can be applied to a wide variety of biomedical devices. Various applications include:

- catheter coatings to increase biocompatibility and lubricity; e.g., urinary tract catheters, coronary catheters, tracheal catheters.
- blood contact device coatings to decrease thrombogenicity; e.g., vascular grafts, shunts, pacemaker leads.
- coatings of subcutaneous or intramuscular implants to minimize fibrous capsule formation; e.g. reconstructive/prosthetic implants, i.e., breast protheses.
- coatings to increase lubricity of the surface; e.g. condoms, pacemaker leads, urinary tract catheters.
- coatings to deliver therapeutics to modify cellular responses and control infection; e.g., pacemaker electrodes and lead bodies for the management of inflammation, defibrillator patch leads and pulse generators for the control of infection.
- lubricous coatings for the delivery of antivirals, spermicides, flavorings or scents; e.g., condoms (male and female).
- a material to be used in the formation of ultraviolet laser induced microcapsules for the controlled release of a variety of therapeutics.

A coating is applied in conventional manners such as by dipping, spraying and brushing. The coating can be effective when applied in thicknesses as thin as 0.01 μm (0.01 microns), but generally it is preferably applied in a thickness of at least 1 μm (1 micron). The thicknesses used herein refer to the thickness before hydration, i.e., before the coating is placed in contact with body fluids.

The *in vivo* implantation of foreign material often interferes with such host processes as normal tissue or organ regeneration and healing. Typically, the host detrimental response to the foreign material is mediated by an inflammatory response. The present invention provides compositions and procedures which lessen the inflammatory response and enhance the healing process in association with the implantation of a foreign device. This effect is achieved by allowing the proliferation of stable cells (i.e., those which possess the ability to replicate, but do not often do so) and permanent cells (i.e., those which lack the ability to replicate). Examples of a stable cell are hepatocytes (liver cells); examples of permanent cells include nerve cells, skeletal muscle cells, and cardiac muscle cells. At the time of injury associated with the implant, these cells respond to the initiation of an inflammatory response such that inflammatory exudate is produced which leads to fibrosis. Inflammation, generally defined as the reaction of vascularized tissue to local injury, serves to contain, neutralize, or block off an injurious agent or process.

One example of a "foreign body" or object which is often associated with a deleterious inflammatory response is the pacing lead electrode of a pacemaker. The contact of a pacing lead electrode of a pacemaker upon insertion into endocardial tissue inflicts injury at the site of contact which results in inflammation, wound healing and other reactions at the insertion site. Typically, these reactions result in the formation of scar tissue surrounding the electrode which can have a deleterious effect on pacing thresholds. Consequently, scar or granulation tissue is physiologically different than endocardial tissue.

Cardiac cells comprising the endocardium are unique in that they can be polarized/depolarized and thus contract or relax with a given stimulus. On the other hand, cells which comprise scar tissue, as well as most other cells, lack this capability. As a result, when scar tissue forms around a foreign object, such as a pacing lead electrode, an environment is created which makes it difficult for the electronic signal to reach the cardiac cells. As a consequence, more energy must be expanded from the pacemaker battery in order for sufficient electrical impulse to penetrate through the scar tissue and reach the healthy endocardial tissue. Thus, the formation of scar tissue around the distal electrode of a pacemaker lead is highly deleterious to the lifespan of the pacemaker.

An approach to limiting the formation of scar tissue would be to introduce an appropriate protein or polypeptide to the environment adjacent to the pacing lead electrode. The presence of an appropriate protein, such as an extracellular matrix molecule, will promote rapid healing of injured endocardium, myocardium, or epicardium. The tissue which results from accelerating the healing process in this manner would contain minimal fibrous capsule and more native parenchymal cells. The benefits from such a response include: (1) rapid tissue growth around the electrode to effectively "anchor" the lead; (2) prevention of chronic inflammation due to abrasion as well as dislodgement; and (3) regeneration of non-granular tissue, resulting in decreased energy requirements from the pacemaker. By inhibiting formation of scar tissue, less electrical energy would have to be utilized to reach the healthy endocardial tissue. As a consequence, it would be possible to achieve enhanced battery life and allow further miniaturization of the pacemaker housing or "can" for the electronics by decreasing battery demand.

Thus, in another embodiment, the invention provides a method for inhibiting the formation of scar tissue at a site by promoting the attachment and growth of viable cells on or into a biocompatible surface of an implantable device, wherein the surface of the device is treated with an extracellular matrix molecule, or fragment thereof, and a biocompatible polymer. The method for attachment and growth of cells on a surface may be performed *in vivo* or *in vitro*. The device having the treated surface can be an electrode, such as the electrode of a pacemaker.

Extracellular matrices which can be used to coat the device include collagen, laminin, fibronectin, vitronectin, merosin, versican, aggrecan, tenascin and fragments thereof as well as integrins, glycoproteins and proteoglycans which make up interstitial connective tissue and basement membranes. Fragments of an extracellular matrix molecule or cell attachment molecule which may be utilized are capable of stimulating the attachment and growth of non-granular tissue at the site of insertion of the device in the host. An example of a fragment useful for such purposes is the RGD peptide (arginine-glycine-aspartic acid) which is a fragment of fibronectin.

The biocompatible polymer useful as a substrate in the method of the invention includes a sulfonated poly(tetrafluoroethylene), such as Nafion™, gelatin, poly(vinylpyrrolidone), poly(acrylamide), and poly(ethylene glycol). Those of skill in the art will know of other acceptable polymers which can be used according to the invention.

The biocompatible polymer coating the surface of the implantable device may further comprise at least one growth factor, which allows proliferation of cells and viable tissue. Examples of such growth factors include epidermal growth factor (EGF), platelet derived growth factor (PDGF) and fibroblast growth factor (FGF). The growth factor can be applied directly to the coating of the device or may be contained in slow-release material which serves as the coating. Those of skill in the art know of such materials or can readily identify and produce such materials, without undue experimentation.

In yet another embodiment, the invention provides a method for preparing a surface of an implantable device, wherein the surface inhibits formation of scar tissue and promotes the attachment and growth of cells to the surface (e.g., the electrode of a pacemaker), which comprises applying a biocompatible polymer and an extracellular matrix molecule or fragment thereof to the surface of a device. The biocompatible polymer on the surface of the device may further comprise at least one growth factor as described above. Application of the biocompatible polymer and extracellular matrix molecule may be by spraying or dipping the surface in the appropriate solution, for example. Preferably the biocompatible polymer is applied to the surface, followed by application of the extracellular matrix protein. However, the extracellular matrix molecule and the biocompatible polymer can be applied concurrently after being dissolved or dispersed into an appropriate solvent. Such a solvent would preferably be a physiological buffer such as a phosphate buffer (pH 7.2-7.6), for example, phosphate buffered saline (PBS). Following application of the biocompatible polymer to the surface of the device, the surface may be further treated with, for example, ultraviolet irradiation which can initiate cross-linking of an appropriate biocompatible polymer, if necessary.

The amount of biocompatible polymer and extracellular matrix coating the surface of the device can vary depending on such factors as the toxicity level of the coating material. The coating depth is influenced by the coating solution concentration (i.e., percent solids, the number of applications and so forth). Preferably, the concentration of the coating material is from about 0.1% to about 10% by weight. Preferred extracellular matrix and/or growth factor concentrations are from about 0.1% to about 10% of total coating weight.

The invention also provides an implantable, stimulating electrode having a surface coated with a polymer and an extracellular matrix molecule or fragment thereof, wherein the coating of the electrode allows attachment and growth of cells on the surface. Preferred is a pacemaker lead wherein the lead portion containing the distal electrode to the proximal ring is coated with the polymer coating.

These and other features of the present invention will become apparent from the following examples.

Example 1

(Preparation of Polymer)

140 g of poly(ethylene oxide) (average molecular weight of 20,000) was dissolved in 1 liter of toluene and the water was azeotropically removed using a Dean Stark trap. The remaining solvent was then removed using a rotary evaporator. 300 ml of dry-tetrahydrofuran was added and the mixture was heated to dissolve the PEG. The reaction was kept under a dry inert atmosphere of nitrogen while stirring until the reaction was completed.

0.504 g of sodium hydride (NaH) was added to the stirring solution and after thirty minutes, 5.78 g of 4-bromomethylbenzophenone was added, and the reaction stirred overnight.

The reaction was sampled intermittently to determine the equivalent weight, before quenching the reaction, by the following procedure. A 5 ml sample of the reaction was treated with 50 µl acetic acid and evaporated. Toluene (10 ml) was added to the residue and removed using a rotary evaporator. The residue was dissolved in toluene (10 ml) and filtered with a 0.45µ syringe filter. The clear toluene solution was added to cold $Et_2O$ with stirring. The solid was collected on a Büchner funnel and reprecipitated from toluene (10 ml) and $Et_2O$ (50 ml). The solid was isolated by filtration. Traces of solvents were removed by agitation of the solid at room temperature under a vacuum of less than 1 mm. A solution of the solvent free modified poly(ethylene oxide) in water was made at a concentration of 0.3 to 0.5 mg/ml. The equivalent weight was calculated from the equation:

$$\text{Eq. Wt.} = [\text{Conc(mg/ml)} * \in(1.802 * 10^4)]/\text{Absorbance(260 nm)}$$

Once the equivalent weight was below 10,500, the reaction was quenched by addition of 2 moles acetic acid per mole of NaH. The quenched reaction was then rotary evaporated to remove the tetrahydrofuran. The residue was dissolved in toluene (1000 ml), and the warm cloudy toluene solution was centrifuged and filtered to remove insolubles. The clear toluene solution was rotary evaporated to remove 700 ml of the toluene. The warm concentrated toluene solution of the product was then added to 1500 ml of cold (0°C) stirred $Et_2O$. The modified poly(ethylene oxide) was isolated on a Büchner funnel, and reprecipitated from toluene (300 ml) and $Et_2O$ (1500 ml). The final weight of the modified poly(ethylene oxide) obtained was 136g (95% of theory).

Example 2

(Preparation of Anti-fouling Coatings)

The following procedure was be used to coat surfaces with the photochemically reactive polymer of Example 1.

Polyurethene tubing to be coated was cleaned by wiping with a solvent such as isopropyl alcohol or other cleaning agent and then treated in plasma to activate its surface. The plasma treatment involved exposing the surface to argon gas plasma at 250 mTorrs pressure for 2 minutes per side at 250 watts power. The plasma treated tubing was then dip-coated in an aqueous solution of the modified poly(alkylene oxide) of Example 1. The solution which can contain polymer in amounts from 50 to 500 mg/ml, with higher concentrations yielding thicker coatings, was used to coat 3 segments of tubing. The dip consisted of a 30-second soak followed by removal of the device at a rate of approximately 70-80 cm/min. Excess polymer on the surface of the device was drained by allowing the tube to hang for about 15 seconds. The wet samples were then exposed to ultraviolet light for about three minutes in an ELC lamp chamber. The ultraviolet light exposure crosslinked the prepolymer to form a tenacious, lubricous, hydrophilic anti-fouling coating on the surface.

Coating thickness was measured on the three samples prepared from three different concentrations of the BBE-$PEG_{20,000}$-BBE of Example 1, 50 mg/ml, 100 mg/ml, and 200 mg/ml. The coating prepared from 50 mg/ml solution yielded a uniform, dry coating with a thickness the order of 0.5 micron, at 100 mg/ml the dry coating thickness was 1.4 microns, and at 200 mg/ml the dry coating thickness was 3 microns.

Example 3

(Lubricity of the Coating)

The coefficient of friction for the anti-fouling coating on the polyurethane tubing of Example 2 was determined by utilizing the "beef casing" method. This method determines the minimum force required to pull a beef casing across the hydrated polymer surface. For the uncoated polyurethane tubing the average force required was 225.20 grams to yield an average coefficient of friction of 1.11. Hydrated coated tubing yielded an average force of 42.04 (grams) and an average coefficient of friction of 0.21. This represents an increase in lubricity

by more than a factor of five over uncoated polyurethane tubing.

Although the present invention has been described in considerable detail with regard to certain preferred versions thereof, other versions are possible. For example, in branch type formulas such as those of Formulas 3, 4, 8, 9, 15, 16, 20, and 21 where there are 4 chains extending from a central carbon atom, or Formulas 5, 10, and 22, where there are 2 or 3 chains extending from a central carbon atom, the chains need not be the same, i.e., the $R_1$'s and $R_2$'s can be different.

Example 4

(Coating the Device)

This example describes various methods which can be used to coat devices which can be implanted *in vivo*. The coatings described enhance the growth of non-granular tissue forming cells and, as a result, induce minimal scarification when implanted in a host. The device used to illustrate the use of these coatings was a pacemaker lead electrode.

METHOD A

Photoderivatized polyethylene glycol (PEG) (1 gram) was solubilized in 5 ml of water with the aid of an orbital shaker to yield a solution with a concentration of 200 mg/ml. The most distal electrode of a pacemaker lead was submerged into a bath of warm iso-propyl alcohol and the lead was agitated to clean the electrode. The electrode was removed and allowed to air dry for 5-10 minutes. The dried electrode was dipped into the PEG solution and the lead was set into a holder with the electrode surface in the upright position. The coating was allowed to set for an additional 5 minutes and the procedure was repeated until the desired coating thickness was achieved. Upon completion of the last dipping procedure this electrode was allowed to fully dry at 50°C and the distal electrode was irradiated at 305 Nm for 5 minutes to affect cross-linking. The lead was removed from the UV chamber and placed in a vacuum oven at 50°C for a period of 1 hour at which time the oven was evacuated to 84.7 kPa (25 inches Hg) for 24 hours to ensure complete dehydration.

Concurrently, 0.01g of peptide arginine-glycineaspartic acid (RGD), Sigma Chemical A 8052, was dissolved in 10 ml of physiological buffer (phosphate buffer, pH 7.0) at room temperature. The electrode with the dehydrated electrode coating was placed into the peptide solution and the coating was allowed to fully hydrate. The electrode was removed from the solution and placed into the holding rack for an hour at room temperature. This procedure was repeated one time. The partially dry coating was placed into a vacuum chamber and allowed to sit at 84.7 kPa (25 inches Hg) for an additional 24 hours to ensure complete dehydration. The lead was exposed to ethylene oxide sterilization (twice) and allowed to aerate.

METHOD B

The photoderivatized PEG (1 gram) was solubilized in 5 ml of water with the aid of an orbital shaker to yield a solution with a concentration of 200 mg/ml. The most distal electrode of a pacemaker lead was submerged into a bath of warm iso-propyl alcohol and the lead was agitated to clean the electrode. The electrode was removed and allowed to air dry for 5-10 minutes. The dried electrode was dipped into the PEG solution and the lead was set into a holder with the electrode surface in the upright position and the lead holder was placed in the UV chamber and the coating irradiated (wet) at 305 Nm for 5 minutes. The procedure was repeated until the desired coating thickness was achieved. The lead was removed from the UV chamber and placed into a vacuum oven at 50 C for a period of 1 hour at which time the oven was evacuated to 84.7 kPa (25 inches Hg) for 24 hours to ensure complete dehydration.

Concurrently, 0.01g of RGD was dissolved in 10 ml of physiological buffer (phosphate buffer, pH 7.0) at room temperature. The electrode with the dehydrated electrode coating was placed into the peptide solution and the coating was allowed to fully hydrate. The electrode was removed from the solution and placed into the holding rack for an hour at room temperature. This procedure was repeated one time. The partially dry coating was placed into a vacuum chamber and allowed to sit at 84.7 kPa (25 inches Hg) for an additional 24 hours to ensure complete dehydration. The lead was exposed to ethylene oxide sterilization (twice) and allowed to aerate.

METHOD C

The photoderivitized PEG (1 gram) was solubilized in water (4 ml) with the aid of an orbital shaker to yield

a solution with a concentration of 250 mg/ml. In a separate container, 0.01 g of the peptide, RGD (0.99% by weight), was solubilized in 1.0 ml of sterile water and the peptide solution was added to the PEG solution and the vial containing the mixture was placed on the orbital shaker until homogeneity was achieved.

The most distal electrode of the pacemaker lead was submerged into a bath of warm isopropyl alcohol and the lead was agitated to clean the electrode. The electrode was removed and allowed to air dry for 5-10 minutes. The dried electrode was dipped into the PEG solution and the lead set into a holder with the electrode surface in the upright position. The coating was allowed to dry for 5 minutes and the procedure repeated until the desired coating thickness was achieved. Upon completion of the last dipping procedure, the electrode was allowed to fully dry at room temperature and the distal electrode was irradiated at 305 Nm for 5 minutes. The lead was removed from the UV chamber and placed into a vacuum oven and evacuated to 84.7 kPa (25 inches Hg) for 24 hours to ensure complete dehydration. The lead was exposed to ethylene oxide sterilization (twice) and allowed to aerate.

METHOD D

The photoderivitized PEG (1 gram) was solubilized in water (4 ml) with the aid of an orbital shaker to yield a solution with a concentration of 250 mg/ml. In a separate container, 0.01 g of the peptide, RGD, was solubilized in 1.0 ml of sterile water and the peptide solution was added to the PEG solution and the vial containing the mixture was placed on the orbital shaker until homogeneity was achieved.

The most distal electrode of the pacemaker lead was submerged into a bath of warm isopropyl alcohol and the lead was agitated to clean the electrode. The electrode was removed and allowed to air dry for 5-10 minutes. The dried electrode was dipped into the PEG solution and the lead set into a holder with the electrode surface in the upright position. The lead holder was placed into a UV chamber in the upright position and the coating was irradiated (wet) for 5 minutes at 305 Nm. The dipping/irradiation procedure was repeated until the desired coating thickness was achieved. Upon completion of the last dipping/irradiation procedure, the lead was removed from the UV chamber and placed into a vacuum oven and evacuated to 84.7 kPa (25 inches Hg) for 24 hours to ensure complete dehydration. The lead was exposed to ethylene oxide sterilization (twice) and allowed to aerate.

## Claims

1. A composition for coating a surface of an implantable device, wherein the composition inhibits formation of scar tissue at an *in vivo* site when the device is inserted at the site, characterised in that the composition comprises a biocompatible polymer and an extracellular matrix molecule or fragment thereof.

2. A composition as claimed in Claim 1, characterised in that the polymer is selected from a sulfonated poly(tetrafluoroethylene), gelatin, poly(vinylpyrrolidone), poly(acrylamide), and poly(ethylene glycol).

3. A composition as claimed in Claim 1 or Claim 2, characterised in that the extracellular matrix molecule is selected from laminin, collagen, fibronectin, vitronectin, merosin, versican, aggrecan and tenascin.

4. A composition as claimed in any of Claims 1 to 3, characterised in that it further contains at least one growth factor.

5. A composition as claimed in Claim 4, characterised in that the growth factor is selected from epidermal growth factor, platelet-derived growth factor and fibroblast growth factor.

6. An electrode, characterised in that it is coated with a composition as claimed in any preceding Claim.

7. An electrode as claimed in Claim 6, characterised in that the electrode is a pacemaker lead electrode.

8. The use of a composition as claimed in any of Claims 1 to 5 in the inhibition of scar tissue formation at an *in vivo* site of insertion of a device, in which a surface of the device is coated with the composition.

9. The use of a composition, as claimed in Claim 8, characterised in that the device is an electrode, such as a pacemaker lead electrode.

10. The use of a composition, as claimed in Claim 8 or Claim 9, characterised in that the site of insertion is the heart.

European Patent Office

# EUROPEAN SEARCH REPORT

Application Number
EP 94 30 4571

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | WO-A-91 05036 (BOARD OF REGENTS, THE UNIVERSITY OF TEXAS SYSTEM) * claims * --- | 1-10 | A61L27/00 |
| P,X | EP-A-0 585 476 (UNITED STATES SURGICAL CORP.) * page 6, line 25 - line 50; examples * --- | 1-5 | |
| X | WO-A-89 11500 (COMMONWEALTH SCIENTIFIC AND INDUSTRIAL RESEARCH ORGANISATION.) * claims * --- | 1-3 | |
| Y | US-A-4 281 669 (DAVID C. MACGREGOR) * claims * --- | 1-10 | |
| Y | WO-A-89 08467 (IMEDEX) * claims * --- | 1-10 | |
| A | WO-A-92 22312 (WADSTRÖM, J.) * claims * --- | 1 | TECHNICAL FIELDS SEARCHED (Int.Cl.5) |
| A | EP-A-0 321 924 (WILL MÖLLER AG.) * claims * ----- | 1 | A61L |

The present search report has been drawn up for all claims

| Place of search | Date of completion of the search | Examiner |
|---|---|---|
| THE HAGUE | 26 October 1994 | ESPINOSA, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)